# EUROPÄISCHE PATENTANMELDUNG

(11) **EP 2 308 891 A1**
(43) Veröffentlichungstag der Anmeldung: **13.04.2011**
(21) Anmeldenummer: 09171335.4
(22) Anmeldetag: 25.09.2009
(51) Int. Cl.: C07K 14/47, C12Q 1/68

(54) **Varianten von STAT-Transkriptionsfaktoren mit verbesserter DNA-Bindung**

(71) Anmelder: PHILIPPS-UNIVERSITÄT MARBURG, 35037 Marburg (DE)
(72) Erfinder: Meyer, Thomas, 34225 Baunatal (DE); Koch, Verena, 35519 Rockenberg (DE); Ruppert, Volker, 35075 Gladenbach (DE); Maisch, Bernhard, 35043 Marburg (DE)
(74) Vertreter: Stumpf, Peter

(57) **Zusammenfassung**

Die Erfindung umfasst die Beschreibung und Verwendung von Gensequenzen und der exprimierten Proteine oder Proteinsequenzen des Zytokin-abhängigen Transkriptionsfaktors STAT1 und der homologen Sequenzen in anderen STAT-Transkriptionsfaktoren, sowie deren Proteinsequenzen mit einer gegenüber den Wildtyp-STAT-Transkriptionsfaktoren erhöhten Affinität zu korrespondierenden DNA-Sequenzen auf Grund einer verbesserten spezifischen Erkennung der korrespondierenden DNA-Sequenzen. Durch die Identifikation des zugrunde liegenden molekularen Mechanismus zur Dissoziation der STAT-Transkriptionsfaktoren von den korrespondierenden DNA-Sequenzen ermöglicht diese Erfindung einen verbesserten Nachweis des Aktivierungszustandes des jeweiligen STAT-Transkriptionsfaktors.

## Beschreibung

Die vorliegende Erfindung betrifft Varianten der zytokin-abhängigen STAT-Transkriptionsfaktoren sowie deren Verwendung. Diese STAT-Transkriptionsfaktoren werden durch deren Aminosäurensequenzen oder Nukleinsäuresequenzen beschrieben und ermöglichen wegen ihrer erhöhten Affinität zu korrespondierenden DNA-Sequenzen eine verbesserte spezifische Erkennung palindromischer DNA-Bindungsstellen.

### Abkürzungen und Definitionen

cDNA: complementary DNA; mittels des Enzyms Reverse Transkriptase aus RNA synthetisierte DNA
DNA: deoxyribonucleic acid, Desoxyribonukleinsäure
Korrespondierende DNA-Sequenz: Genbindungsstelle der Doppelstrang-Oligonukleotide; spezifische palindromische Gen- bzw. DNA-Bindungsstellen bzw. - sequenzen in der Promotorregion eines Genes, an denen die STAT-Transkriptionsfaktoren binden
Jak: Januskinase; Tyrosinkinasen im Zytoplasma von Zellen
RNA: ribonucleic acid, Ribonukleinsäure
SH2-Domäne: Src-homology-2-Domäne; vermittelt die Bildung von STAT-Dimeren durch Tyrosin-Phosphorylisierung
Src: Gruppe von zytoplasmatischen Tyrosinkinasen
STAT: signal transducer and activator of transcription, Signal-Transduktor und Aktivator der Transkription; werden auch als STAT-Proteine oder STAT-Transkriptionsfaktoren bezeichnet
Vektor: Transportvehikel zur Übertragung einer fremden Nukleinsäure (beispielsweise DNA) in eine lebende Empfängerzelle

### Beschreibung des allgemeinen Gebietes der Erfindung

Eine Vielzahl verschiedener Transkriptionsfaktoren ist an der Umsetzung des genetischen Codes in Struktur- oder Funktionsproteine beteiligt. Dazu müssen die Transkriptionsfaktoren bestimmte Bereiche in der Promotorregion der korrespondierenden DNA-Sequenz erkennen können und daran binden. Die Stärke dieser Bindung hängt von der Struktur des betreffenden Transkriptionsfaktors ab, das heißt, von seiner Aminosäurensequenz. Daraus folgt, dass eine Änderung der Aminosäurensequenz eines Transkriptionsfaktors dessen Bindungsfähigkeit an der korrespondierenden DNA-Sequenz verändern kann.

Eine Gruppe dieser Transkriptionsfaktoren wird als STAT-Transkriptionsfaktoren bezeichnet, wobei sieben verschiedene, natürlich vorkommende STAT-Transkriptionsfaktoren (Wildtyp-Varianten der STAT-Transkriptionsfaktoren) bei Säugetieren, inklusive dem Mensch, bekannt sind (STAT1, STAT2, STAT3, STAT4, STAT5a, STAT5b, STAT6). Die Aminosäurensequenzen dieser sieben Wildtyp-Varianten der STAT-Transkriptionsfaktoren sind dem Fachmann bekannt. STAT-Transkriptionsfaktoren sind Proteine, welche eine hochkonservierte Familie von homologen Transkriptionsfaktoren bilden, die nach Stimulation durch extrazelluläre Zytokine vielfältige Einflüsse auf die Genexpression ausüben. STAT-Transkriptionsfaktoren sind beispielsweise an der Regulierung des Immunsystems, des Zellwachstums und der Zellproliferation beteiligt.

Die Bindung extrazellulärer Zytokine aktiviert zytoplasmatische Janus-Kinasen (Jak), die spezifische Tyrosinreste in Zytokin-Rezeptoren und STAT-Transkriptionsfaktoren phosphorylieren. STAT-Transkriptionsfaktoren können darauf hin durch ihre SH2-Domäne mit dem Phosphotyrosin-Rest des kontralateralen Monomers dimerisieren und als dimere STAT-Transkriptionsfaktoren in den Zellkern transportiert werden, wo durch Bindung an die korrespondierenden DNA-Sequenzen eine Gentranskription initiiert wird. Diese über Jak und STAT vermittelte Genaktivierung ist ein vorübergehender Prozess, da die STAT-Transkriptionsfaktoren im Zellkern durch kernständige Tyrosin-Phosphatasen inaktiviert werden. Über diese Inaktivierung der STAT-Transkriptionsfaktoren und deren Lösung von den korrespondierenden DNA-Sequenzen ist wenig bekannt.

Zellen mit abweichend hoher und/oder verlängerter Jak- oder STAT-Aktivierung sind anfällig für Transformationen und assoziiert mit abnormaler Entwicklung. Beispielsweise können bestimmte STAT-Transkriptionsfaktoren und die von ihnen gesteuerten Zielproteine an der Entstehung von Tumorerkrankungen, bei Defekten des Immunsystems oder bei Stoffwechselerkrankungen beteiligt sein.

### Stand der Technik

STAT-Transkriptionsfaktoren vermitteln die intrazelluläre Wirkung von Zytokinen wie beispielsweise Interferonen oder Interleukinen, indem sie die korrespondierenden DNA-Sequenzen erkennen und an ihnen binden. Da diese Zytokine als Angriffspunkte für Antitumor-Medikamente und Immunsuppressiva fungieren, werden die STAT-Transkriptionsfaktoren als pharmakologische Zielstrukturen zum Screening von neuen Wirkstoffen oder Medikamenten eingesetzt. Die Messung der spezifischen DNA-Bindung von STAT-Transkriptionsfaktoren an den korrespondierenden DNA-Sequenzen gilt als einfach durchzuführendes und zugleich hochsensitives Verfahren für die Bestimmung der pharmakologischen Wirkstärke eines Wirkstoffes oder Medikamentes mit tumor- oder entzündungshemmender Wirkung, die über STAT-Transkriptionsfaktoren wirken. In diesem Verfahren macht man sich die hohe Affinität und Sequenzspezifität der Bindung der STAT-Transkriptionsfaktoren an der korrespondierenden DNA-Sequenz zu nutze. Durch eine gelelektrophoretische Bestimmung der Affinität von STAT-Transkriptionsfaktoren zu spezifischen korrespondierenden DNA-Sequenzen wird direkt die Dauer und Intensität der STAT-Aktivierung gemessen oder es werden fluoreszenzmikroskopische Verfahren für eine indirekte Messung eingesetzt.

### Nachteile des Standes der Technik

Bisher wurden lediglich die Wildtyp-Varianten der STAT-Transkriptionsfaktoren in Testverfahren zur Beurteilung der Wirksamkeit von neuen Wirkstoffen oder Medikamenten mit tumor- oder entzündungshemmender Wirkung eingesetzt. Auf Grund zu niedriger Affinität der Wildtyp-Varianten für die korrespondierenden DNA-Sequenzen ist es trotz des Einsatzes von sensitiven Testverfahren oft nicht möglich, die sequenzspezifische DNA-Bindung von STAT-Transkriptionsfaktoren zu detektieren (Detektionsproblem). Damit ist auch die Wirksamkeitsprüfung für neue Wirkstoffe oder Medikamente nicht mehr möglich.

STAT-Transkriptionsfaktoren mit einer gegenüber den Wildtyp-Varianten der STAT-Transkriptionsfaktoren deutlich erhöhten Affinität eignen sich in idealer Weise für solche pharmakologischen Testungen und sind den Wildtyp-Varianten der STAT-Transkriptionsfaktoren signifikant überlegen. Bislang sind solche Varianten der Wildtyp-Varianten der STAT-Transkriptionsfaktoren nicht bekannt.

### Aufgabe

Aufgabe der Erfindung ist erstens die Bereitstellung von Varianten der STAT-Transkriptionsfaktoren, welche eine gegenüber den Wildtyp-Varianten der STAT-Transkriptionsfaktoren deutlich erhöhte Bindungsaffinität mit erhaltener Erkennung der korrespondierenden DNA-Sequenzen aufweisen.

Zweitens soll unter Verwendung der erfindungsgemäßen STAT-Transkriptionsfaktoren ein Verfahren beschrieben werden, welches geeignet ist, in der pharmakologischen Wirkstoffforschung die Wirksamkeit neuer Wirkstoffe oder Medikamente nachzuweisen und somit das Detektionsproblem zu lösen.

### Lösung

Die Aufgabe wird erfindungsgemäß gelöst gemäß den Ansprüchen.

Durch die Identifizierung eines molekularen Mechanismus zur Dissoziation der STAT-Transkriptionsfaktoren von der korrespondierenden DNA-Sequenz konnten neuartige Mutanten von STAT-Transkriptionsfaktoren entwickelt werden, welche wegen ihres Dissoziationsverhaltens einen verbesserten Nachweis der spezifischen DNA-Bindung an einer korrespondierenden DNA-Sequenz ermöglichen und somit eine technische Lösung für das Detektionsproblem zur Verfügung stellen.

Es werden erfindungsgemäß veränderte Varianten der STAT-Transkriptionsfaktoren sowie die korrespondierenden cDNA- und Proteinsequenzen zur Verfügung gestellt, welche im Vergleich zu den Wildtyp-Varianten der STAT-Transkriptionsfaktoren eine deutlich erhöhte Bindungsaffinität aufweisen. Die beiden in der DNA-Bindedomäne der STAT1-Variante der STAT-Transkriptionsfaktoren befindlichen Glutaminsäure-Reste in den Positionen 411 und 421 sind für das Bindeverhalten der STAT-Transkriptionsfaktoren essentiell, da deren endständigen Carboxylatgruppen für die Dissoziation der STAT-Transkriptionsfaktoren von den korrespondierenden DNA-Sequenzen benötigt werden. Die Substitution dieser negativ geladenen Aminosäuren gegen neutrale oder positiv geladene Aminosäuren, wie beispielsweise Alanin oder Lysin bewirkt eine verminderte Inaktivierung durch kernständige Tyrosin-Phosphatasen und damit eine erhöhte Bindungsaffinität der betreffenden erfindungsgemäß veränderten STAT-Transkriptionsfaktoren an den korrespondierenden DNA-Sequenzen. Damit wird eine verminderte Dissoziationsrate erreicht, welche zu einer verstärkten Bindung der erfindungsgemäß veränderten STAT-Transkriptionsfaktoren an den korrespondierenden DNA-Sequenzen führt. Durch die Verwendung dieser erfindungsgemäßen Varianten der STAT-Transkriptionsfaktoren lässt sich das Detektionsproblem lösen und somit Untersuchungen zur Wirksamkeit neuer Wirkstoffe oder Medikamente technisch verbessern.

Die erfindungsgemäßen STAT-Transkriptionsfaktoren sind entweder gentechnisch durch rekombinante Nukleinsäuren oder durch multiple Synthese herstellbar. Ihre Herstellung erfolgt durch dem Fachmann bekannte biosynthetische oder chemische Verfahren.

### Ausführungsbeispiele

### Beispiel 1:

Ersatz der Glutaminsäure-Reste in den Positionen 411 und/oder 421 im Wildtyp-STAT1-Molekül oder homologer Aminosäurereste in anderen Mitgliedern der STAT-Familie durch positiv geladene Aminosäure-Reste.

### Beispiel 2:

Ersatz der Glutaminsäure-Reste in den Positionen 411 und/oder 421 im Wildtyp-STAT1-Molekül oder homologer Aminosäurereste in anderen Mitgliedern der STAT-Familie durch neutral geladene Aminosäure-Reste.

Ein besonders vorteilhaftes Ausführungsbeispiel zum Ausführungsbeispiel 1 ist das

### Beispiel 3:

Ersatz der Glutaminsäure-Reste in den Positionen 411 und/oder 421 im Wildtyp-STAT1-Molekül oder homologer Aminosäurereste in anderen Mitgliedern der STAT-Familie durch Alanin-Reste.

Ein besonders vorteilhaftes Ausführungsbeispiel zum Ausführungsbeispiel 2 ist das

### Beispiel 4:

Ersatz der Glutaminsäure-Reste in den Positionen 411 und/oder 421 im Wildtyp-STAT1-Molekül oder homologer Aminosäurereste in anderen Mitgliedern der STAT-Familie durch Lysin-Reste.

Für alle Ausführungsbeispiele gilt, dass die erfindungsgemäßen STAT-Transkriptionsfaktoren als Monomer, Dimer oder Polymer vorliegen können.

Für alle Ausführungsbeispiele gilt, dass die erfindungsgemäßen STAT-Transkriptionsfaktoren auch durch die für sie kodierenden Nukleinsäuresequenzen beschrieben werden. Besonders vorteilhaft für die Beschreibung der erfindungsgemäßen STAT-Transkriptionsfaktoren sind cDNA-Sequenzen des Volle-Länge-Moleküls oder trunkierte Deletionsvarianten davon.

Für alle Ausführungsbeispiele gilt, dass die für die erfindungsgemäßen STAT-Transkriptionsfaktoren kodierende Nukleinsäuresequenzen mit Hilfe eines Vektors in die Zielzelle eingebracht werden.

Für alle Ausführungsbeispiele gilt, dass die erfindungsgemäßen STAT-Transkriptionsfaktoren zum Nachweis von aktiven Substanzen eingesetzt werden, welche die Transkriptionsaktivität von Zytokin-responsiven Zielgenen modifizieren. Insbesondere gilt dies für Interleukin- oder Interferon-responsive Zielgene.

Für alle Ausführungsbeispiele gilt, dass die erfindungsgemäßen STAT-Transkriptionsfaktoren zur Bestimmung der pharmakologischen Wirkstärke eines Wirkstoffes oder Medikamentes eingesetzt werden. Besonders vorteilhaft ist der Einsatz der erfindungsgemäßen STAT-Transkriptionsfaktoren zum Screening von Wirkstoffen oder Medikamenten, welche die Transkriptionsaktivität von Zytokin-responsiven Zielgenen beeinflussen. Dies gilt insbesondere für den Einsatz der erfindungsgemäßen STAT-Transkriptionsfaktoren zum Screening von Wirkstoffen oder Medikamenten, welche für die Diagnostik oder Therapie von Krankheiten eingesetzt werden, die mit einer veränderten STAT-Transkriptionsfaktorenbindung einhergehen.

## Patentansprüche

1. STAT-Transkriptionsfaktoren, **dadurch gekennzeichnet, dass** mindestens einer der für die Dissoziation von den korrespondierenden DNA-Sequenzen benötigten Aminosäure-Reste im Bereich der DNA-Bindungsdomäne der Wildtyp-STAT-Transkriptionsfaktoren mutiert ist.

2. STAT-Transkriptionsfaktoren nach Anspruch 1, **dadurch gekennzeichnet, dass** die Aminosäure Glutaminsäure in den Positionen 411 und/oder 421 im Wildtyp-STAT1-Transkriptionsfaktor oder homologe Aminosäurereste in anderen Mitgliedern der STAT-Familie mutiert sind.

3. STAT-Transkriptionsfaktoren nach Anspruch 2, **dadurch gekennzeichnet, dass** die Aminosäure Glutaminsäure in den Positionen 411 und/oder 421 im Wildtyp-STAT1-Transkriptionsfaktor oder homologe Aminosäurereste in anderen Mitgliedern der STAT-Familie durch positiv geladene oder neutrale Aminosäurereste ersetzt sind.

4. STAT-Transkriptionsfaktoren nach Anspruch 3, **dadurch gekennzeichnet, dass** die Aminosäure Glutaminsäure in den Positionen 411 und/oder 421 im Wildtyp-STAT1-Transkriptionsfaktor oder homologe Aminosäurereste in anderen Mitgliedern der STAT-Familie durch Alanin-Reste ersetzt sind.

5. STAT-Transkriptionsfaktoren nach Anspruch 3, **dadurch gekennzeichnet, dass** die Aminosäure Glutaminsäure in den Positionen 411 und/oder 421 im Wildtyp-STAT1-Transkriptionsfaktor oder homologe Aminosäurereste in anderen Mitgliedern der STAT-Familie durch Lysin-Reste ersetzt sind.

6. STAT-Transkriptionsfaktor-kodierende Nukleinsäuresequenzen, bevorzugt cDNA-Sequenzen des Volle-Länge-Moleküls oder trunkierte Deletionsvarianten davon, **dadurch gekennzeichnet, dass** sie anstatt des Aminosäure-Restes Glutaminsäure in den Positionen 411 und/oder 421 im Wildtyp-STAT1-Transkriptionsfaktor oder homologer Aminosäurereste in anderen Mitgliedern der STAT-Familie für positiv geladene oder neutrale Aminosäuren-Reste kodieren, bevorzugt für Alanin-Reste oder Lysin-Reste.

7. Proteinsequenzen die durch eine Nukleinsäuresequenz nach Anspruch 6 kodiert werden, **dadurch gekennzeichnet, dass** die Aminosäure Glutaminsäure in den Positionen 411 und/oder 421 im Wildtyp-STAT1-Transkriptionsfaktor oder homologe Aminosäurereste in anderen Mitgliedern der STAT-Familie durch positiv geladene oder neutrale Aminosäure-Reste ersetzt sind, bevorzugt durch Alanin-Reste oder Lysin-Reste.

8. STAT-Transkriptionsfaktoren nach Anspruch 1, **dadurch gekennzeichnet, dass** sie in monomerer, dimerer oder polymerer Form vorliegen.

9. Ein Vektor, **dadurch gekennzeichnet, dass** er eine Nukleinsäuresequenz nach Anspruch 6 beinhaltet.

10. Die Verwendung der STAT-Transkriptionsfaktoren nach Anspruch 1 zum Nachweis von aktiven Substanzen, welche die Transkriptionsaktivität von Zytokin-responsiven Zielgenen modifizieren.

11. Die Verwendung der STAT-Transkriptionsfaktoren nach Anspruch 1 zur Bestimmung der pharmakologischen Wirkstärke eines Wirkstoffes oder Medikamentes.

12. Verfahren zum Screening von Wirkstoffen oder Medikamenten, welches die Transkriptionsaktivität von Zielgenen unter Verwendung der Ansprüche 1 bis 11 untersucht.

13. Verfahren zum Screening von Wirkstoffen oder Medikamenten nach Anspruch 12, **dadurch gekennzeichnet, dass** die erfindungsgemäßen STAT-Transkriptionsfaktoren an den korrespondierenden DNA-Sequenzen der Zielgene binden.

14. Verfahren zum Screening von Wirkstoffen oder Medikamenten nach Anspruch 13, **dadurch gekennzeichnet, dass** die aktiven Substanzen zur Diagnostik oder Therapie von Krankheiten eingesetzt werden, welche mit einer veränderten STAT-Transkriptionsfaktorenbindung einhergehen.
